# EUROPEAN PATENT SPECIFICATION

(11) **EP 1 088 096 B1**
(45) Date of publication and mention of the grant of the patent: **12.10.2005**
(21) Application number: 99907122.8
(22) Date of filing: 18.02.1999
(51) Int. Cl.: C12Q 1/00, A01N 63/00, C07H 21/02, G01N 33/50, C12N 5/06

(54) **METHODS OF SCREENING COMPOUNDS FOR BIOACTIVITY IN ORGANIZED TISSUE**
VERFAHREN ZUM SCREENEN VON VERBINDUNGEN MIT BIOAKTIVITÄT IN ORGANGEWEBEN
PROCEDES DE CRIBLAGE DE COMPOSES DESTINES A RECHERCHER UNE BIOACTIVITE DANS UN TISSU STRUCTURE

(30) Priority: 18.02.1998 US 75054; 22.05.1998 US 86370
(43) Date of publication of application: 04.04.2001
(73) Proprietor: Vandenburgh, Herman, H., Providence, RI 02906 (US); Valentini, Robert F., Cranston, RI 02905 (US)
(72) Inventor: VANDENBURGH, Herman, H., Providence, RI 02506 (US); VALENTINI, Robert, F., Cranston, RI 02905 (US)
(74) Representative: Maschio, Antonio
(86) International application number: PCT/US1999/003459
(87) International publication number: WO 1999/042604

(56) References cited:
- WO-A-96/40175
- WO-A-97/36615
- US-A- 4 940 853
- US-A- 5 153 136
- US-A- 5 869 041
- VANDENBURGH H ET AL: "TISSUE-ENGINEERED SKELETAL MUSCLE ORGANOIDS FOR REVERSIBLE GENE THERAPY" HUMAN GENE THERAPY, vol. 7, no. 17, 10 November 1996 (1996-11-10), pages 2195-2200, XP000652998 ISSN: 1043-0342
- DERMER G.B., "Another Anniversary of the War on Cancer", BIO/TECHNOLOGY, 12 March 1994, Vol. 12, page 320, XP002920341
- DATABASE SCISEARCH, No. 91:170241, VANDENBURGH et al., "Insulin and IGF-1 Induce Promounced Hypertrophy of Skeletal Myofibers in Tissue-Culture", AM. J. PHYSIOL., 1991, Vol. 260, No. 3, pp. C475-C484, XP002920342

## Description

### Background of the Invention

The invention relates to the screening of candidate compounds for bioactivity in a tissue. One of the primary therapies used to treat disease is the delivery of bioactive compounds to an affected organism.

*In vitro* screening of compounds for biological activity has been disclosed in the prior art as assays, for example, in which monolayers of tissue cultured cells are exposed to a candidate compound and a biological response in the cells is measured. For example, monolayers of disorganized muscle fibers have been shown to respond to anabolic growth factors. See Vandenburgh et al. (Vandenburgh et al., Am. J. Physiol. 260: C475-C484, 1991) which discloses induction of hypertrophy of skeletal muscle myofibers by insulin and insulin-like growth factors. See Janeczko et al. (Janeczko et al., J. Biol. Chem. 259: 6292-6297, 1984) which discloses that multiplication-stimulating activity inhibits intracellular proteolysis in muscle monolayer cultures. See Vandenburgh et al. (Vandenburgh et al., Am. J. Physiol. 259: C232-C240, 1990) which discloses modulation of protein degradation and synthesis by prostaglandins in muscle monolayer cultures. *In vivo* methods of compound screening also have been performed in animals to test the biological response of a host tissue (Dupont et al., J. Appl. Physiol. 80: 734-741, 1996).

WO 96/40175 discloses a culture system wherein cells are grown on a three dimensional framework. In WO 97/36615, smooth muscle cells are grown in monolayer culture in vitro. This document does not teach the use of an organized tissue. VANDENBURGH H ET AL: 'TISSUE-ENGINEERED SKELETAL MUSCLE ORGANOIDS FOR REVERSIBLE GENE THERAPY' HUMAN GENE THERAPY, vol. 7, no. 17, 10 November 1996 (1996-11-10), pages 2195-2200, ISSN: 1043-0342 discloses the subcutaneous implantation of tissue-engineered skeletal muscle organoids for gene therapy purposes. Before implantation, these organoids are grown and maintained in culture plates.

Tissue-cultured cells of primary tissue have been utilized for testing of compounds *in vitro.* A chief disadvantage of such primary cell cultures is their relatively short-term viability *in vitro* (about 7-14 days) in the differentiated state (Volz et al., J. Mol. Cell. Cardiol. 23, 161-173, 1991). Most cell types in a two-dimensional, monolayer culture system (e.g. skeletal muscle, cardiac muscle, fibroblasts, bone and cartilage) dedifferentiate within about 14 days. In addition, certain cell types (e.g. muscle, fibroblasts, bone and cartilage) are anchorage dependent and when these adherent cells grown as a monolayer are spontaneously released into the culture medium they will die.

Monolayer tissue-cultured cells of primary tissue are, in many instances, unsuitable for testing of a compound because they do not differentiate as fully as in vivo cells.

Muscle cells, in particular, must fuse in order to differentiate. However, muscle cells grown in a two-dimensional culture system fuse in a disorganized manner that does not reflect the organization of the tissue of origin and do not fully differentiate.

There is a need in the art for methods of screening for a biologically active compound which permit the production of tissue from cells that have fused or coalesced in an organized manner similar to the tissue of origin; for example the organization and morphology of muscle tissue may include parallel arrays of striated muscle tissue. These cells are more fully differentiated than disorganized muscle tissue and would better predict in vitro, the response of the cells in vivo to bioactive compounds.

There is also a need in the art for methods of screening for a biologically active compound which permit long-term *in vitro* tissue maintenance in the differentiated state.

There also is a need in the art for methods of screening for a biologically active compound which permit transfer of a tissue between in vivo and *in vitro* environments wherein the tissue does not undergo any substantial structural and/or shape changes during the transfer step and wherein the tissue can be transferred more than once.

There is also a need in the art for methods of screening for a biologically active compound which permits administering the biologically active compound by injection into the bulk of an organized tissue; wherein the step of injection is similar to the step of injecting a subject.

There is also a need in the art for methods of screening for a biologically active compound which permits sensing in a tissue through changes in overall size, length or shape.

There is also a need in the art for methods of screening for a biologically active compound which permits using an organized tissue as a sensor for changes in biological parameters.

### Summary of the Invention

In general, the invention features a method of screening a compound for bioactivity in an organized tissue. The method includes the steps of contacting a candidate bioactive compound with an organized tissue and measuring in at least one or more cells of the organized tissue, a biological parameter that is associated with bioactivity wherein a change in the biological parameter that occurs as a result of the contacting step is indicative of bioactivity of the candidate compound.

In a preferred embodiment of this method, the step of contacting comprises exposing a candidate bioactive compound to an organized tissue comprising substantially post-mitotic cells.

In other preferred embodiments, the step of contacting comprises contacting exposing a candidate bioactive compound to an organized tissue comprising cells aligned substantially parallel to each other and along a dimension of the vessel in which the cells were grown.

In other preferred embodiments, the organized tissue is comprised of cells wherein at least a subset of the cells contain a heterologous gene.

In other preferred embodiments, the cells containing the heterologous gene produce a substance of a type that is not normally present in the cells or in an amount that is not normally produced by the cells.

In other preferred embodiments, the contacting step comprises exposing a candidate bioactive compound to an organized tissue comprising muscle cells.

In other preferred embodiments the substantially post-mitotic cells of the organized tissue are muscle cells.

In other preferred embodiments, cells aligned substantially parallel to each other are muscle cells.

In other preferred embodiments, the biological parameter comprises protein degradation.

In other preferred embodiments, the heterologous gene is a reporter gene encoding a detectable protein.

In other preferred embodiments, the measuring step comprises sensing a detectable protein.

In a related aspect, the invention also features a method of screening a compound for bioactivity, comprising the steps of administering a candidate bioactive compound to a host organism in which an organized tissue is implanted wherein administration is selected from the group consisting of oral consumption, tissue absorption via topical compositions, via suppositories and via inhalants; removing at least a subset of cells of the organized tissue from the host organism; and measuring in at least a subset of cells of the organized tissue a biological parameter that is associated with bioactivity, wherein a change in the biological parameter that occurs as a result of the contacting step is indicative of bioactivity of the candidate compound.

In a related aspect, the invention also features a method of screening a compound for bioactivity, comprising the steps of administering a candidate bioactive compound to a host organism in which an organized tissue is implanted wherein administration is selected from the group consisting of oral consumption, tissue absorption via topical compositions, via suppositories and via inhalants; and measuring in at least one or more cells of the organized tissue a biological parameter that is associated with bioactivity, wherein a change in the biological parameter that occurs as a result of said contacting step is indicative of bioactivity of said candidate compound. The organized tissue of the invention could produce a marker or a product encoded for by a foreign DNA.

In a related aspect, the invention also features a method of screening a compound for bioactivity, comprising the steps of administering a candidate bioactive compound to a host organism in which an organized tissue is implanted wherein administration is selected from the group consisting of oral consumption, tissue absorption via topical compositions, via suppositories and via inhalants; and measuring in a host organism a biological parameter that is associated with bioactivity, wherein a change in the biological parameter that occurs as a result of said contacting step is indicative of bioactivity of said candidate compound. The organized tissue of the invention could produce a marker or a product encoded for by a foreign DNA

In a related aspect, the invention also features a method of screening a compound for bioactivity, comprising the steps of transplanting an organized tissue into a host organism wherein the organized tissue produces a bioactive compound in the organism; and measuring a biological parameter in the host organism, wherein alteration of the biological parameter that occurs as a result of implantation of the organized tissue and production of the bioactive compound is indicative of the biological activity of the compound. The organized tissue of the invention could produce a marker or a product encoded for by a foreign DNA

In a preferred embodiment of this method, the measuring step is performed on a body fluid sample of the host organism.

The invention also features a kit for performing a method in accordance with the invention comprising a plurality of organized tissues wherein each organized tissue is contained in a container.

In a preferred embodiment of the kit the container comprises a culture plate containing a plurality of wells, wherein each well contains an organized tissue or said plurality of organized tissues in medium and under conditions wherein the organoid is viable, long-term.

Further features and advantages of the invention are as follows. The organized tissue of the claimed invention provides a more *in vivo*-like culture system for screening the activity of biological compounds and offers significant advantages over disorganized tissue. For example, poorly differentiated cells respond differently to compounds as compared to organized cells *in vivo*. This invention also provides methods for screening a bioactive compound in a tissue which reflects the *in vivo* cellular organization and gross morphology of the natural *in vivo* tissue. This organized tissue system offers a more efficient and accurate method for screening candidate bioactive compounds for desired biological effects *in vitro* and in vivo, and permits screening on a long-term rather than a short-term basis.

Further features and advantages of the invention will become more fully apparent in the following description of the embodiments and drawings thereof, and from the claims.

### Description

The invention provides for in vitro and in vivo screening methods in which a candidate biologically active ("bioactive") compound may be screened for its biological effects on an organized tissue or a cell or cells of an organized tissue, or a host organism in which the tissue is implanted.

As used herein, by "bioactive compound" is meant a compound which influences the biological structure, function, metabolism, or activity of a cell or tissue of a living organism. The candidate bioactive compound will not include the medium or an undefined (i.e., unidentified) component of the medium in which the tissue is tested. The medium may be serum containing or serum-free, as described herein. A component of the medium may be one or more of the following: serum, salt (ions), vitamins, water, selenium, and chicken embryo extract). Preferably, the candidate bioactive compound will consist essentially of the compound to be tested, and this will not include the medium in which the tissue is cultured.

"Biological parameter" refers to a measurable characteristic of a biological process of a tissue, cell or organism that is "associated with" a bioactivity and includes but is not limited to measurable chemical changes (e.g. ions, proteins, ATP, receptors), measurable mechanical changes (e.g. force, size, shape, contractile status) or measurable electrical changes (membrane potential, ion flux, electrical output). For example, the biological parameters of protein degradation, cell damage marker production, and ubiquitination levels are measured to indicate the bioactivity (biological process, for example protein synthesis or creatine kinase release) of muscle wasting. Alternatively, the biological parameters of growth factor production are measured to indicate the biosynthetic and secretory activity of muscle cells. Alternatively, the biological parameters of glucose and lactate production are measured to indicate the metabolic activity of muscle cells.

"Associated with" refers to art-accepted scientific correlation between a biological parameter and a biological activity; that is, the biological parameter is what is measured that indicates biological activity.

By "organized tissue" or "organoid" is meant a tissue formed *in vitro* from a collection of cells having a cellular organization and gross morphology similar to that of the tissue of origin for at least a subset of the cells in the collection. An organized tissue, as used herein, does not include a scaffold which is a pre-formed solid support that imparts or provides short-term (hours to 2 weeks in culture) structure or support to the tissue or is required to form the tissue. An organized tissue or organoid may include a mixture of different cells, for example, muscle (including but not limited to striated muscle, which includes both skeletal and cardiac muscle tissue), fibroblast, and nerve cells, and may exhibit the *in vivo* cellular organization and gross morphology that is characteristic of a given tissue including at least one of those cells, for example, the organization and morphology of muscle tissue may include parallel arrays of striated muscle tissue. Preferably the organized tissue will include cells that are substantially post-mitotic, and/or aligned substantially parallel to each other and along a given axis of the three-dimensional tissue (with the tissue having x,y and z axes). In an organized tissue with fibers oriented in a lengthwise manner, the length of the organized tissue is about 2mm - 20cm (x, y) and more then 1cell layer thick (z). It is preferred that the length of the organized tissue is in the range of about 10mm - 100 mm (x, y) and 0.05mm to 2.0mm thick (z). In contrast, a monolayer of cells is typically on the order of 1-10 µm in thickness. Preferably, the organized tissue will have contraction signaling properties. By "contraction signaling properties" is meant an ability to generate a directed force by changes in overall size, length, and shape.

By "*in vivo*-like gross and cellular morphology" is meant a three-dimensional shape and cellular organization substantially similar to that of the tissue *in vivo.*

Cell types from which an organized tissue is formed include but are not limited to muscle (smooth and striated), bone, cartilage, tendon, nerve, endothelial and fibroblast.

By "three-dimensional" is meant an organized tissue having x, y and z axes wherein x and y of the axes are at least 2 mm with z at least 0.05 mm thick, and wherein 1, 2 or all of the axes are as great as 20 cm. Preferably, a three-dimensional tissue can be transferred between *in vivo* and *in vitro* environments multiple times without undergoing any substantial structural and/or shape changes. More preferably a three dimensional tissue can be injected by a method wherein the bulk of a three-dimensional tissue is injected. More preferably, a three-dimensional tissue is capable of contraction signaling. By "contraction signaling" is meant the ability to generate a directed force by changes in overall size, length, and shape. More preferably, a three-dimensional tissue can be used as a sensor for changes in biological parameters. Preferably a three-dimensional muscle tissue is comprised of cells that have fused in an organized manner similar to the tissue of origin; for example the organization and morphology of muscle tissue may include parallel arrays of striated muscle tissue.

By " unorganized tissue" is meant cells show little in vivo like intercellular relationship to each other.

By "at least a subset of cells of an organized tissue" is meant at least two cells, preferably at least 10% of the cells of the tissue, and more preferably at least 25% of the cells.

By "substantially post-mitotic cells" is meant an organoid in which at least 50% of the cells are non-proliferative. Preferably, organoids including substantially post-mitotic cells are those in which at least 80% of the cells are non-proliferative. More preferably, organoids including substantially post-mitotic cells are those in which at least 90% of the cells are non-proliferative. Most preferably, organoids including substantially post-mitotic cells are those in which 99% of the cells are non-proliferative. Cells of an organoid retaining proliferative capacity may include cells of any of the types included in the tissue. For example, in striated muscle organoids such as skeletal muscle organoids, the proliferative cells may include muscle stem cells (i.e., satellite cells) and fibroblasts.

By "aligned substantially parallel" is meant aligned substantially parallel to each other and along a given axis of the three-dimensional tissue, which is preferably the longest axis of the tissue (with the tissue having x,y and z axes).

By "substantially all of the cells" is meant at least 90% and preferably 95-99% of the cells.

According to the methods of the invention, a host organism may be implanted with an organized tissue. Since substantially all of the aligned/organized cells comprising the organized tissue are differentiated, migration of these cells to other anatomical sites is reduced. Moreover, implantation of post-mitotic, non-migratory myofibers reduces the possibility of cell transformation and tumor formation.

By "monolayer" is meant a single cell layer.

By "differentiated" is meant cells with numerous mature-like characteristics, either chemical or physical.

By "terminally differentiated" is meant is not capable of further proliferation or differentiation into another cell or tissue.

By "migrating cell" is meant that the cell is released from the organized tissue so as to be viable in an environment that is not physically associated with the organized tissue, whether in vivo or in vitro.

By "non-migrating cells " is meant that the cell is not released from the organized tissue.

By " substantially all of the cells of the organized tissue are non-migrating" is meant that at least 50% and preferably 80%, 90% and most preferably 98-100% of the cells of a given tissue type are non-migrating.

By " of a type that is not normally present in the cells" is meant foreign to the cell.

By "in an amount that is not normally produced by the cells" is meant at least 5% above or below the amount normally produced by the cells or tissue preferably at least 10% above or below, more preferably 50-100% above or below, or greater than 100% above the amount normally produced by the cells or tissue.

By "heterologous gene" is meant a DNA sequence that is introduced into a cell.

By "foreign DNA sequence" is meant a DNA sequence which differs from that of the wild type genomic DNA of the organism and may be extra-chromosomal, integrated into the chromosome, or the result of a mutation in the genomic DNA sequence.

By "muscle wasting" is meant a loss of muscle mass due to reduced protein synthesis and/or accelerated breakdown of muscle proteins, including for example, as a result of activation of the non-lysosomal ATP-ubiquitin-dependent pathway of protein degradation.

By "attenuation of muscle wasting " is meant preventing or inhibiting muscle wasting.

By "short-term" is meant a length of time in which cells are viable for a period that does not exceed but includes 14 days.

By "long-term" is meant a length of time in which cells are viable that is more than 14 days and as long as 30 days, 60 days and 90 days or more.

By "body fluid" is meant serum, saliva, lymphatic fluid, urine and the like.

"Contacting" refers to exposing the tissue, or cells thereof, to a compound, or mixing the tissue and the compound.

According to the invention, any "change" in a biological parameter refers to alterations (i.e. an increase or decrease) from a steady state level (for example protein degradation, creatine kinase release, heat shock promoter activity, second messenger activity, growth factor production, glucose and lactate production) of the parameter in a tissue subjected to the candidate bioactive compound. Such a change is indicative of bioactivity. The level of inhibition (decrease) or enhancement (increase) will be at least 1% per hour (or per day or per month as appropriate), preferably 10% and more preferably at least 50-100%, (or 10-fold, and 50-100 fold, as applicable) from greater or less than the level measured in the absence of the candidate compound.

### SCREENING METHODS

A method of screening a candidate compound for bioactivity in a tissue includes culturing an organized tissue in the presence or absence of a candidate bioactive compound, and measuring a biological parameter of the tissue or one or more cells of the tissue.

A candidate bioactive compound may be screened in an organized tissue comprised, for example, of muscle cells. A biological parameter measurable in muscle tissue, and of interest in the invention is, for example, muscle wasting and attenuation of muscle wasting.

Muscle wasting is a loss of muscle mass due to reduced protein synthesis and/or accelerated breakdown of muscle proteins, largely as a result of activation of the non-lysosomal ATP-ubiquitin-dependent pathway of protein degradation. Muscle wasting is caused by a variety of conditions including cachexia associated with diseases including various types of cancer and AIDS, febrile infection, denervation atrophy, steroid therapy, surgery, trauma and any event or condition resulting in a negative nitrogen balance. Muscle wasting also occurs following nerve injury, fasting, fever, acidosis and certain endocrinopathies.

### Production of an organized tissue

An organized tissue is produced for use in the invention as described in U.S. Patent Numbers: 4,940,853 and 5,153,136. In addition, an organized tissue may be produced as follows.

### In Vitro Production of Tissues Having In Vivo-like Gross and Cellular Morphology

Organized tissues having *in vivo*-like gross and cellular morphology may be produced *in vitro* from the individual cells of a tissue of interest. As a first step in this process, disaggregated or partially disaggregated cells are mixed with a solution of extracellular matrix components to create a suspension. This suspension is then placed in a vessel having a three dimensional geometry which approximates the *in vivo* gross morphology of the tissue and includes tissue attachment surfaces coupled to the vessel. The cells and extracellular matrix components are then allowed to coalesce or gel within the vessel, and the vessel is placed within a culture chamber and surrounded with media under conditions in which the cells are allowed to form an organized tissue connected to the attachment surfaces.

By "extracellular matrix components" is meant compounds, whether natural or synthetic compounds, which function as substrates for cell attachment and growth. Examples of extracellular matrix components include, without limitation, collagen, laminin, fibronectin, vitronectin, elastin, glycosaminoglycans, proteoglycans, and combinations of some or all of these components (e.g., Matrigel™, Collaborative Research, Catalog No. 40234).

By "tissue attachment surfaces" is meant surfaces having a texture, charge or coating to which cells may adhere *in vitro.* Examples of attachment surfaces include, without limitation, stainless steel wire, VELCRO™, suturing material, native tendon, covalently modified plastics (e.g., RGD complex), and silicon rubber tubing having a textured surface.

Although this method is compatible with the *in vitro* production of a wide variety of tissues, it is particularly suitable for tissues in which at least a subset of the individual cells are exposed to and impacted by mechanical forces during tissue development, remodeling or normal physiologic function. Examples of such tissues include muscle, bone, skin, nerve, tendon, cartilage, connective tissue, endothelial tissue, epithelial tissue, and lung. More specific examples include skeletal and cardiac (i.e., striated), and smooth muscle, stratified or lamellar bone, and hyaline cartilage. Where the tissue includes a plurality of cell types, the different types of cells may be obtained from the same or different organisms, the same or different donors, and the same or different tissues. Moreover, the cells may be primary cells or immortalized cells. Furthermore, all or some of the cells of the tissue may contain a foreign DNA sequence (for example a foreign DNA sequence encoding a receptor) which indicates the response to a bioactive compound (as described herein).

The composition of the solution of extracellular matrix components will vary according to the tissue produced. Representative extracellular matrix components include, but are not limited to, collagen, laminin, fibronectin, vitronectin, elastin, glycosaminoglycans, proteoglycans, and combinations of some or all of these components (e.g., Matrigel™, Collaborative Research, Catalog No. 40234). In tissues containing cell types which are responsive to mechanical forces, the solution of extracellular matrix components preferably gels or coalesces such that the cells are exposed to forces associated with the internal tension in the gel.

An apparatus for producing a tissue *in vitro* having an *in vivo*-like gross and cellular morphology includes a vessel having a three dimensional geometry which approximates the *in vivo* gross morphology of the tissue. The apparatus also includes tissue attachment surfaces coupled to the vessel. Such a vessel may be constructed from a variety of materials which are compatible with the culturing of cells and tissues (e.g., capable of being sterilized and compatible with a particular solution of extracellular matrix components) and which are formable into three dimensional shapes approximating the *in vivo* gross morphology of a tissue of interest. The tissue attachment surfaces (e.g., stainless steel mesh, VELCRO™, or the like) are coupled to the vessel and positioned such that as the tissue forms *in vitro* the cells may adhere to and align between the attachment surfaces. The tissue attachment surfaces may be constructed from a variety of materials which are compatible with the culturing of cells and tissues (e.g., capable of being sterilized, or having an appropriate surface charge, texture, or coating for cell adherence).

The tissue attachment surfaces may be coupled in a variety of ways to an interior or exterior surface of the vessel. Alternatively, the tissue attachment surfaces may be coupled to the culture chamber such that they are positioned adjacent to the vessel and accessible by the cells during tissue formation. In addition to serving as points of adherence, in certain tissue types (e.g., muscle), the attachment surfaces allow for the development of tension by the tissue between opposing attachment surfaces. Moreover, where it is desirable to maintain this tension *in vivo,* the tissue attachment surfaces may be implanted into an organism along with the tissue.

A vessel for producing an organized tissue that is suitable for the *in vitro* production of a skeletal muscle organoid, has a substantially semi-cylindrical shape and tissue attachment surfaces coupled to an interior surface of the vessel (Shansky et al., In Vitro Cell Develop. Biol. 33: 659-661, 1997).

### In Vitro Production of a Skeletal Muscle organoid having In Vivo-Like Gross and Cellular Morphology

Using an apparatus and method as generally described above, a skeletal muscle organoid having an *in vivo*-like gross and cellular morphology was produced *in vitro.* During skeletal muscle development embryonic myoblasts proliferate, differentiate, and then fuse to form multi-nucleated myofbers. Although the myofibers are non-proliferative, a population of muscle stem cells (i.e., satellite cells), derived from the embryonic myoblast precursor cells, retain their proliferative capacity and serve as a source of myoblasts for muscle regeneration in the adult organism. Therefore, either embryonic myoblasts or adult skeletal muscle stem cells may serve as one of the types of precursor cells for *in vitro* production of a skeletal muscle organoid.

To produce skeletal muscle organoids, primary avian, rat or human muscle stem cells or immortalized murine muscle cells, were suspended in a solution of collagen and Matrigel™ which was maintained at 4°C to prevent gelling. The cell suspension was then placed in a semi-cylindrical vessel with tissue attachment surfaces coupled to an interior surface at each end of the vessel. The vessel was positioned in the bottom of a standard cell culture chamber. Following two to four hours of incubation at 37°C, the gelled cell suspension was covered with fresh culture medium (renewed at 24 to 72 hour intervals) and the chamber containing the suspended cells was maintained in a humidified 5% CO₂ incubator at 37°C throughout the experiment.

Between the second and sixth day of culture, the cells were found to be organized to the extent that they spontaneously detached from the vessel. At this stage, the cells were suspended in culture medium while coupled under tension between tissue attachment surfaces positioned at either end of the culture vessel. During the subsequent ten to fourteen days, the cells formed an organoid containing skeletal myofibers aligned parallel to each other in three dimensions. The alignment of the myofibers and the gross and cellular morphology of the organoid were similar to that of *in vivo* skeletal muscle.

To carry out the above method, an apparatus for organoid formation was constructed from silastic tubing and either VELCRO™ or metal screens as follows. A section of silastic tubing (approximately 5 mm I.D., 8 mm O.D., and 30 mm length) was split in half with a razor blade and sealed at each end with silicone rubber caulking. Strips of VELCRO™ (loop or hook side, 3 mm wide by 4 mm long) or L-shaped strips of stainless steel screen (3 mm wide by 4 mm long by 4 mm high) were then attached with silicone rubber caulking to the interior surface of the split tubing near the sealed ends. The apparatus was thoroughly rinsed with distilled/deionized water and subjected to gas sterilization.

Skeletal muscle organoids were produced *in vitro* from a C2C12 mouse skeletal muscle myoblast cell line stably co-transfected with recombinant human growth hormone-expressing and b-galactosidase-expressing (b-gal) constructs (Dhawan et al., 1991, *Science* 254:1509-1512) or from primary avian myoblasts or from primary rat myoblasts (both neonatal and adult cells) or from primary human myoblasts (both fetal and adult satellite cells).

Cells were plated in the vessel at a density of 1-4 X 10⁶ cells per vessel in 400 µl of a solution containing extracellular matrix components. The suspension of cells and extracellular matrix components was achieved by the following method. The solution includes 1 part Matrigel™ (Collaborative Research, Catalog No. 40234) and 6 parts of a 1.6 mg/ml solution of rat tail Type I collagen (Collaborative Research, Catalog No. 40236). The Matrigel™ was thawed slowly on ice and kept chilled until use. The collagen solution was prepared just prior to cell plating by adding to lyophilized collagen, growth medium (see constituents below), and 0.1N NaOH in volumes equivalent to 90% and 10%, respectively, of the volume required to obtain a final concentration of 1.6 mg/ml and a pH of 7.0-7.3. The collagen, sodium hydroxide and growth medium were maintained on ice prior to and after mixing by inversion.

Freshly centrifuged cells were suspended in the collagen solution by trituration with a chilled sterile pipet. Matrigel™ was subsequently added with a chilled pipet and the suspension was once again mixed by trituration. The suspension of cells and extracellular matrix components was maintained on ice until it was plated in the vessel using chilled pipet tips. The solution was pipetted and spread along the length of the vessel, taking care to integrate the solution into the tissue attachment surfaces. The culture chamber containing the vessel was then placed in a standard cell culture incubator, taking care not to shake or disturb the suspension. The suspension was allowed to gel, and after 2 hours the culture chamber was filled with growth medium such that the vessel was submerged.

Skeletal muscle organoids were produced from adult human biopsied skeletal muscle by the following method. Standard muscle biopsies were performed on two adult male volunteers and myoblasts isolated by standard tissue culture techniques (Webster et al., 1990, *Somatic Cell and Mol. Gen.* 16:557-565). One hundred muscle stem cells (myoblasts) were identified from each biopsy by immunocytochemical staining with an antibody against desmin and the myoblasts were expanded through at least 30 doubling. The 100 myoblasts could thus be expanded into greater than 50 billion cells (5 x 10¹⁰).

Skeletal muscle cells were cultured into organoids according to the following conditions. For a period of three days the cells were maintained on growth medium containing DMEM-high glucose (GIBCO-BRL), 5% fetal calf serum (Hyclone Laboratories), and 1% penicillin/streptomycin solution (final concentration 100 units/ml and 0.1 mg/ml, respectively). On the fourth day of culture, the cells were switched to fusion medium containing DMEM-high glucose, 2% horse serum (Hyclone Laboratories), and 100 units/ml penicillin for a period of 4 days. On the eighth day of culture, the cells were switched to maintenance medium containing DMEM-high glucose, 10% horse serum, 5% fetal calf serum, and 100 units/ml penicillin for the remainder of the experiment. In certain embodiments cells were maintained in a defined serum-free medium containing insulin, transferrin and selenium. Before the organoids were ready for implantation, some were cultured in maintenance media containing 1 mg/ml of cytosine arabinoside for the final four to eight days. Treatment with cytosine arabinoside eliminated proliferating cells and produced organoids containing substantially post-mitotic cells. The growth medium can be replaced manually or automatically by a perfusion system.

### Use of Foreign DNA as a Marker for Screening Bioactive Compounds

An organoid useful in the invention may produce a substance in an amount or of a type not normally produced by the cells or tissue in response to a bioactive compound (i.e. that can be measured, for example, a marker compound). In this embodiment, at least some of the cells of the organoid contain a foreign DNA sequence. The foreign DNA sequence may be extra-chromosomal, integrated into the genomic DNA of the organoid cell, or may result from a mutation in the genomic DNA of the organoid cell. In addition, the cells of the organoid may contain multiple foreign DNA sequences. Moreover, the different cells of the organoid may contain different foreign DNA sequences. For example, in one embodiment, a skeletal muscle organoid may include myofibers containing a first foreign DNA sequence and fibroblasts containing a second foreign DNA sequence. Alternatively, the skeletal muscle organoid could include myoblasts from different cell lines, each cell line expressing a foreign DNA sequence encoding a different marker compound. These "mosaic" organoids allow the combined and/or synergistic effects of particular bioactive compounds to be measured. For example, myoblasts expressing a detectable growth hormone coupled to a foreign DNA sequence of interest may be combined with myoblasts expressing green fluorescent protein or luciferase coupled to a foreign DNA sequence of interest to produce organoids expressing two detectable markers one secreted and, an additional marker, fluorescent or otherwise, of another cellular function.

In a preferred embodiment, the foreign DNA sequence encodes a protein which is sensitive to a bioactive compound or a substance that is measured as a biological parameter according to the invention. The protein is produced by the cells and liberated from the organoid. Alternatively, the DNA sequence may encode an enzyme or a cell surface protein which mediates sensitivity to a bioactive compound; or a detectable protein encoded by a reporter gene. The DNA sequence may also encode a DNA binding protein which regulates the transcription of the sequence responding to a bioactive compound or an anti-sense RNA which regulates translation of the mRNA responsive to a bioactive compound. The DNA sequence may also bind trans-acting factors, or direct the expression of a factor which may bind trans-acting factors, such that the transcription of the sequence (i.e., foreign or native) is responsive to a bioactive compound (e.g., by disinhibition). Furthermore, the foreign DNA sequence may be a cis-acting control element such as a promoter or an enhancer coupled to a native or foreign coding sequence responsive to a bioactive compound or for an enzyme which mediates the response to a bioactive compound. Thus, the foreign DNA sequence may be expressible in the cell type into which it is introduced and may encode a protein which is synthesized and which may be secreted by such cells. Alternatively, the foreign DNA sequence may be an element that regulates an expressible sequence in the cell. Alternatively, the foreign DNA sequence may encode for a receptor specific for certain classes of molecules or a ligand of a particular class of molecules, that is expressed at a level substantially above or below the normal, endogenous level of expression.

### IN VITRO CULTURE CONDITIONS FOR SCREENING ASSAYS ACCORDING TO THE INVENTION

Culture conditions for screening will vary according to the tissue produced. Methods for culturing cells are well known in the art and are described, for example, in Skeletal Cell Culture: A Practical Approach, (R.I. Fveshney, ed. IRL Press, 1986). In general, the vessel containing the organoid is placed in a standard culture chamber (e.g., wells, dishes, or the like), and the chamber is then filled with culture medium until the vessel is submerged. The composition of the culture medium is varied, for example, according to the tissue produced, the necessity of controlling the proliferation or differentiation of some or all of the cells in the tissue, the length of the culture period and the requirement for particular constituents to mediate the production of a particular bioactive compound. The culture vessel may be constructed from a variety of materials in a variety of shapes as described.

For a varying period (e.g., 3 days) the cells were maintained on growth medium containing DMEM-high glucose (GIBCO-BRL), 5% fetal calf serum (Hyclone Laboratories), and 1% penicillin/streptomycin solution (final concentration 100 units/ml and 0.1 mg/ml, respectively). The growth medium can be replaced manually or automatically by a perfusion system.

### METHOD OF MEASURING THE ACTIVITY OF A CANDIDATE BIOACTIVE COMPOUND IN VITRO

The activity of a candidate compound is determined by measuring a biological parameter that is associated with bioactivity. A change in the biological parameter in the presence of the candidate compound in comparison to the absence of the compound is indicative of activity of the compound.

The activity of a candidate bioactive compound in an organized tissue comprised of muscle cells, can be determined, for example, by measuring muscle wasting in vitro. A number of methods can be used to measure muscle wasting in vitro.

Muscle wasting can be detected by measuring protein synthesis and or degradation, the level of production of cell damage markers such as creatine kinase, the activity of a heat shock protein promoter, and changes in the level of components of the ubiquitin dependent protein degradation pathway.

### 1) Protein Degradation

Total protein degradation increases during muscle atrophy and decreases during attenuation of muscle atrophy. See Vandenburgh et al. Supra. Therefore, inhibition of muscle wasting can be assessed by pulse-chase isotopic methods for measuring the level of radioactive amino acids that are released from a labeled protein. This value is then used as a measure of protein degradation (Vandenburgh and Kaufman., J. Biol. Chem., 255: 5826-5833, 1980).

Replicate samples of an organized tissue comprised of muscle cells will be produced as described above. The organized tissue will be cultured in the presence or absence of a candidate bioactive compound.

Total skeletal muscle protein content is determined by the colorimetric method as described in Chromiak and Vandenburgh, Am. J. Physiol. Cell Physiol., 262: C1471-C1477, 1992. Total skeletal protein content is determined using the bicinchoninic acid (BCA) protein assay (Pierce, Rockford, IL), following extraction of the sample in 0.02 N NaOH overnight at room temperature (Lowry et al., J. Biol. Chem., 139: 795-804, 1941).

Protein synthesis rates are determined by [³H]phe incorporation into cellular proteins. A solution containing 1.25 to 2.5 mCi [³H]phe (sp. act. 57 Ci/mmol; Amersham) is added to the culture medium (either manually or automatically via a perfusion system). After a 6 to 48 hr incubation with continued incubation or perfusion with low serum medium, organoids will be recovered and rinsed extensively with ice-cold Earle's Balanced Salt Solution on a rotary shaker at 120rpm. The organoids are sonicated in 1.0 ml ice-cold sucrose buffer (0.25M sucrose and 0.02M KCI, pH 6.8) in order to determine whether [³H]phe incorporation was linear over this time period. An aliquot of the cell sonicate is made 5% (v/v) with ice-cold trichloroacetic acid (TCA). After 30 min at 4°C, the sonicate is centrifuged for 10 min at 3,000 g at 4°C. The radioactivity in the supernatant [TCA-soluble disintegrations per minute (DPMs)] is measured with a Packard 460C Scintillation Counter. The precipitate is rinsed three times with 5% TCA, the pellet is dissolved in 0.1 N NaOH, and an aliquot is counted for determination of TCA-precipitable (ppt) DPMs. Protein synthesis rates are expressed as TCA ppt DPMs/µg total noncollagenous protein.

For measurement of protein degradation rates, muscle organoids are incubated in medium containing 5.0 µCi/ml [¹⁴C]phe (sp. act. 479 µCi/µmole, Amersham) for 48 hours with a change to fresh medium containing [¹⁴C]phe after the initial 24 hours. After the organoids are rinsed twice over 15 minutes, fresh medium is added either manually or automatically by perfusion. Five hundred µl aliquots of medium are collected every 24 h for measurement of TCA soluble [¹⁴C]phe released from the cells into the medium. A 100 µl aliquot of the medium is mixed with an equal volume of 20% TCA. After 30 min at 4°C, the solution is centrifuged at 3,000 g at 4°C for 10 min. TCA-soluble DPMs in the supernatant are measured. [¹⁴C]phe remaining in the cells at the end of the experiment is determined as outlined above for measuring [¹⁴C]phe in the cellular proteins. The total DPMs incorporated during the initial 48h labeling period, and the TCA soluble DPMs released at each time point are determined, and protein half-lives calculated as T_{½} =(In 2)/*k*, where *k* is the fraction of protein degraded per hour.

The ability of candidate bioactive compounds to modulate the level of degradation of myofibrillar proteins, which constitute 60% of the muscle mass, can also be used as a measure of muscle atrophy. One of the major features of denervation atrophy is differential loss of myofibrillar proteins (Furuno et al., J. Biol. Chem., 265:8550-8557, 1990). The breakdown of these proteins can be followed by measuring 3-methyl-histidine production, which is a specific constituent of actin, and in certain muscles of myosin (Goodman, Biochem. J, 241:121-12, 1987 and Lowell, et al., Metabolism, 35:1121-112, 1986; Stein and Schluter, Am. J. Physiol. Endocrinol. Metab. 272: E688-E696, 1997). When these proteins are hydrolyzed, this amino acid cannot be reutilized in protein synthesis, and thus its appearance as an indication of myofibrillar protein breakdown (Goodman, Biochem. J, 241:121-127, 1987 Lowell et al., Metabolism, 35:1121-112, 1986). The increased production of 3-methyl-histidine after denervation is markedly inhibited by blocking ATP production, but is not affected by treatments that prevent lysosomal and Ca²⁺ -dependent proteolysis (Furuno et al., J. Biol. Chem., 265:8550-8557, 1990; US Patent, 5,340,736).

The degradation of myofibrillar components can be monitored by an assay that measures the release of 3-methylhistidine from muscle proteins (Goodman, Biochem. J, 241:121-12, 1987 and Lowell, et al., Metabolism, 35:1121-112, 1986; Stein and Schluter, Am. J. Physiol. Endocrinol. Metab. 272: E688-E696, 1997, Thompson et al., Am. J. Physiol. 270: C1875-C1879, 1996, Thompson et al., J. Cell. Physiol. 166: 506-511, 1996). Medium aliquots will be removed at various times (2-90 days) after addition of pg to mg amounts of a bioactive compound and 3-methylhistidine measured by HPLC or an amino acid analyzer.

A particularly useful approach to testing the effect of candidate bioactive compounds on the ATP-ubiquitin-dependent degradative process is to administer the candidate bioactive compound to cultured cells in which a short-lived protein whose degradation is ubiquitin-dependent is produced. Modulation of the process of the process of degradation will lead to a change in the level of the protein in the cytosol, as compared to untreated cells. The level of the protein in the cytosol or in the culture medium can be determined, using known methods. For example, cells producing a short-lived enzyme, that is cytosolic, secreted or is engineered to be secreted by the addition of the appropriate signal sequence, can be cultured in the presence or absence of a candidate bioactive compound and the amount of enzyme assayed. Accumulation of the enzyme in the presence of a candidate bioactive compound is indicative of inhibition of the ATP-ubiquitin-dependent process by the candidate bioactive compound being tested. A gene encoding a short-lived protein whose degradation is ubiquitin dependent (e.g., a short-lived enzyme, such as a mutant beta-galactosidase with an abnormal amino terminus targeting it for rapid, ubiquitin-dependent degradation) can be used for this purpose. This recombinant form of beta -galactosidase from *E. coli,* has a half-life of approximately 15 minutes and is degraded by a ubiquitin-dependent pathway (Bachmair et al., Science 234:179-186, 1986; Gonda, et al., J.Biol. Chem., 264:16700-16712 1989). Other mutant forms of enzymes which are rapidly degraded can also be used.

### 2) Monitoring of Markers of Cell Damage

Other methods for quantitating muscle wasting or it's attenuation include comparing the level of release of creatine kinase (a cell damage marker) (Jackson, et al., Neurology, 41: 101-104, 1991) in the presence or absence of the candidate bioactive compound. Creatine kinase activity released by the cells into the culture medium is measured with a commercially available kit (Sigma, Procedure #47-UV). An aliquot of medium is mixed with 1 ml creatine kinase reagent, and the absorbance resulting from production of NADH measured at 340 nm for 5 min at 37° C. The rate of development of absorbance is directly proportional to creatine kinase activity.

### 3) Reporter Gene Expression

Muscle wasting can also be assessed by measuring the activity of a reporter gene, for example, luciferase (LUC) or secreted alkaline phosphatase (SEAP), in cell cultures expressing a construct comprising a heat shock protein promoter directing the expression of a reporter gene of interest.

pUbB-LUC is a vector comprising the luciferase gene under the control of a 1.39 kb fragment of the human ubiquitin B (UbB) promoter (Louis Ferland, unpublished). This promoter fragment contains several copies of the heat shock element (HSE) as well as other regulatory sequences, and is stress responsive. Alternatively, a stress-responsive reporter construct can be made by cloning repeats of the heat shock element NGAAN (Cunniff et al., Mol. Cell. Biol. 11:3504-3514, 1991) upstream of a minimal, enhancerless promoter, for example, the thymidine kinase (tk) proximal promoter, or the Simian Virus 40 (SV40) early promoter, driving the expression of the reporter gene.

Prior to formation of an organized tissue, cells are stably transfected with a plasmid containing the reporter gene under the control of a stress-responsive promoter. An organized tissue comprising cells containing this plasmid is produced as described. Replicate samples of this organized tissue are cultured in the presence or absence of a candidate bioactive compound for various time periods. If the LUC reporter gene is used, LUC activity is measured in aliquots of total cell extract by a chemiluminescent assay using luciferin-ATP as substrate. If the SEAP reporter gene (Clontech) is used, SEAP activity is measured in aliquots of the culture medium, either by a chemiluminescent assay using the CSPD chemiluminescent substrate (Clontech), of by a fluorescent assay using 4-methylumbelliferyl phosphate (MUP) as substrate (Clontech). Alternatively, if the presence of these substrates is not detrimental to the cultured tissues, these substrates are added directly into the culture wells and chemiluminescence or fluorescence measured in the medium by mounting the entire culture vessel (multiwell dish) into a chemiluminescence or fluorescence reader.

Muscle wasting can also be measured by measuring the level of rhGH in the serum of muscle cells expressing a construct comprising the hGH gene under the control of the human ubiquitin B promoter or other heat shock protein promoters.

### 4) Measuring Activity of the Ubiquitin-Dependent Proteolytic System

During muscle atrophy there is an increase in the level of some of the proteins involved in the ubiquitin dependent protein degradation pathway, as well as an increase in the level of mRNA specific for some of these proteins. For example, in skeletal muscles, upregulation of ubiquitin mRNA appears to be a regulated event during muscle wasting. Therefore, since the ubiquitin dependent proteolytic pathway is activated during skeletal muscle atrophy, changes in the level of ubiquitin mRNA and protein in the presence of a candidate compound can be measured and used as a measure of muscle wasting attenuation. Similarly, changes in the level of ubiquitinated proteins in the presence of a candidate compound can be quantitated and used as a measure of muscle wasting attenuation.

### Measuring Ubiquitin mRNA Levels

Replicate samples of organized tissues comprised of muscle cells are produced (as described) and cultured in the presence or absence of a candidate bioactive compound. Total RNA from muscle cells of the organized tissue is isolated by the acid guanidinium isothiocyanate/phenol/chloroform method, and electrophoresis of RNA is performed in 1% agarose gels containing 0.2M formaldehyde (Chomczynski and Sacchi, Anal. Biochem., 162; 156-159, 1987). The RNA is transferred from the gel to nylon membrane (Gene Screen Dupont, NEN Research Pro.) in 20 X SSC (3M sodium chloride/0.3M sodium citrate). RNA is crosslinked to the membrane by UV light at 1200 microjoules on a Stratalinker apparatus (Stratagene Co., CA). Membranes are hybridized at 65 °C with ³²P-labeled cDNA probes prepared by the random-primer method (Feinherg, Vogelstein, Anal. Biochem., 132: 6-13 1983). The hybridization buffer will contain polyvinylpyrrolidone-40,000 (0.2%), Ficoll-400,000 (0.2%), bovine-serum albumin (BSA, 0.2%), Tris-HCl(0.05M, pH 7.5), NaCl (1M), sodium pyrophosphate (0.1%), sodium dodecyl sulfate (SDS, 1%) and salmon sperm DNA (100 mg/ml). After hybridization, the filters are washed in 0.5 X SSC/1% SDS at 42° C. or 65°C. Membranes are exposed to XAR-2 film (Kodak) for autoradiography.

For dot blot analysis, four different concentrations (2-fold dilutions from 1.5 mg) of total denatured RNA from the muscle cells of the organized tissue are spotted onto Gene Screen membranes. The amount of RNA applied to each dot is brought to 1.5 mg by adding *E. coli* tRNA (U.S. Patent 5,340,736).

The hybridization probes will be a Ub cDNA fragment (Agell, et al., Proc. Natl. Acad. Sci. USA, 85:3693-3697, 1988). Blots are hybridized with the Ub probe at 65°C and washed at 65°C. Levels of polyUb RNA are determined from the dot intensities of the autoradiograms by automated densitometric scanning.

### Measuring Ubiqitin System Protein Levels

Total ubiquitin content (which includes both free Ub and Ub ligated to proteins), and other members of the ubiquitin system are measured by the immunochemical method described by Riley et al., (1988) (Riley et al., J. Histochem. Cytochem., 36:621-632, 1988).

Immunoprecipitations are performed on a tissue extract with relevant antibody coupled to protein A-Sepharose, as previously described (Matthews et al., Proc. Natl. Acad. Sci. USA 86:2597-2601, 1989). Control immunoprecipitations with isotype matched, irrelevant monoclonal antibodies are performed. The monoclonal antibodies 2-24 against several subunits of the purified human liver proteasome are available (K. Tanaka and A. Ichihara, University of Tokushima, Japan). Polyclonal antibodies against purified human liver proteasome are also available (see also Matthews et al., Proc. Natl. Acad. Sci. USA, 86:2597-2601, 1989). For immunoblotting, proteins are electrophoresed on a 10% SDS-polyacrylamide gel (Laemmli, U. K. Nature (London) 227:680-685, 1970). After transferring the proteins to nitrocellulose sheets, (Hershko et al., Proc. Natl. Acad. Sci. USA, 77: 1783-1786, 1980) immunoblots are performed as previously described (Hough et al., J. Biol. Chem., 262:8303-8313, 1987; Hough et al., in Ubiquitin (Rechsteiner, M., ed.) pp. 101-134, Plenum Press, New York (1988).

The activity of a candidate bioactive compound in an organized tissue comprised of muscle cells can also be determined by performing biochemical assays that assess muscle cell secretory and metabolic activity.

### 5) Medium Glucose and Lactate Analysis

Glucose and lactate concentrations in the medium can be measured at various intervals over time to determine metabolic activity.

Culture medium samples for biochemical analyses are withdrawn sterilely each day. Glucose utilization and lactate production by the muscle organoids are measured on 500 µl aliquots of medium with a YSI Glucose/Lactate Analyzer Model 2000 (Yellow Springs Instruments, Yellow Springs, OH). Each sample is analyzed in duplicate. The precision of measurement for both glucose and lactate is 0.04 g/L.

### 6) Prostaglandin F_{2α}

Prostaglandin PGF_{2α} is an important anabolic autocrine/paracrine growth factor in skeletal muscle that has been implicated in the stimulation of protein synthesis (Rodemann and Goldberg, J. Biol. Chem., 257: 1632-1638, 1982 and Vandenburgh et al., supra). Its secretion rate is regulated by muscle tension both *in vivo* (Symons et al., J. Appl. Physiol., 77: 1837-1842) and *in vitro* (Vandenburgh et al., supra).

PGF_{2α} production is determined by enzyme immunoassay (EIA, Cayman Chemical Co., Ann Arbor, MI) on culture medium collected every 24 hours (Vandenburgh et al., supra). Sensitivity of the PGF_{2α} EIA at 22°C and 80% binding (B)/initial binding (B0) is 24 pg/ml. The cross-reactivity of the PGF_{2α} antibody is 100% for PGF_{1α}, 7.0% for PGD2, 2.0% for 6-keto PGF_{1α}, 0.3% for 2,3-dinor-6-keto PGF_{1α}, and <0.1% for all other eicosanoids including PGE₂.

### 7) Insulin-Like Growth Factor-1 (IGF-1).

IGF-1 is produced and secreted by skeletal muscle cells, and is an important autocrine/paracrine growth factor in this tissue (Perrone et al., J. Biol. Chem. 270: 2099-2106, 1995).

IGF-1 is determined by a radioimmunoassay (RIA) procedure described previously (Perrone et al., supra). Briefly, media samples are incubated with an equal volume of 0.5 N HCl for 1 hour, and added to C-18 Sep-Pak columns (Millipore, Bedford, MA). Following elution of IGF-binding proteins with 4% acetic acid, IGF-1 is eluted with methanol. Samples are dried in a Savant Speed-Vac (Savant Instruments, Holbrook, NY) and reconstituted in RIA buffer (200 mg/L protamine sulfate, 30 mM NaH₂PO₄H₂O, 0.05% Tween 20, 0.02% (w/v) sodium azide, 0.01 M EDTA), and adjusted to pH 7.5. Samples are incubated with anti-rabbit IGF-1 primary antibody (National Institute of Diabetes and Digestive and Kidney Diseases, National Hormone and Pituitary Program) at 4°C for 24 hr. ¹²⁵I-IGF-1 tracer (Amersham, Arlington Heights, IL), 20,000 cpm per sample, is added and incubated at 4°C for 16 h. IGF-1-primary antibody complexes are precipitated by addition of donkey anti-rabbit antibody (Amersham) and magnetically separated for 15 min. The supernatant is decanted, and radioactivity in the pellet is determined with a Berthold Multi-Crystal Gamma Counter Model LB2104. IGF-1 standards are from Intergen Co. (Purchase, NY). This method can reproducibly detect 12 to 1,000 pg of IGF-1 standard.

### 8) Protein, DNA, and Myosin Heavy Chain (MHC) Content.

To measure protein, DNA and MHC content, the organoids are detached from the wells, transferred to microcentrifuge tubes and frozen at -20°C. The organoids are thawed and sonicated in 1.0 ml ice-cold sucrose buffer (0.25 M sucrose, 0.02 M KCI, pH 6.8), and aliquots removed for determination of total protein, total noncollagenous protein, total DNA and myosin heavy chain (MHC) content as described in Chromiak and Vandenburgh, Am. J. Physiol. Cell Physiol., 262: C1471-C1477, 1992. Noncollagenous protein contents are determined using the bicinchoninic acid (BCA) protein assay (Pierce, Rockford, IL), following extraction of the sample in 0.02 N NaOH overnight at room temperature and removal of collagenous proteins by centrifugation at 3,000g for 10 min (Lowry et al., J. Biol. Chem., 139: 795-804, 1941). Total DNA is determined fluorometrically with Hoescht-33258 with a minor modification of the procedure (Labarca et al., Anal. Biochem., 102: 344-352, 1980). This modification involves eliminating EDTA from the sonication buffer but including it in the phosphate buffer used in the second step of the assay. This is necessary so that an aliquot of the sonicate can also be used for MHC quantitation.

MHC is isolated by gel electrophoresis on 6% polyacrylamide gels. An aliquot of the cell sonicate is mixed with SDS buffer so that final reagent concentrations are 2% sodium dodecyl sulfate, 5% β-mercaptoethanol, 20% glycerol and 0.0625 M Tris-HCl, pH 6.8. Samples are boiled for 5 min before addition to the gel lanes. Electrophoresis is at 160 mV for 90-105 min. Following electrophoresis, gels are fixed in water/methanol/ acetic acid, 45/45/10 (v/v/v), and the protein bands visualized with Fast Stain (Zoion Research, Worcester, MA). Gels are scanned using a CCD camera interfaced with a video monitor and an IBM-compatible computer using JAVA™ image analysis software (Jandel Scientific, San Rafael, CA). MHC quantity is derived by comparing sample band density with a standard curve of band densities for known MHC standards (range of 0.25 µg to 2.0 µg) included on the same set of gels. Calculation of MHC content is made with PEAKFIT™ software (Jandel Scientific). Samples can be retrieved manually as desired or through an on-line system for continuous, programmed monitoring.

### 9) Sensors

An organized tissue of the invention can be used as a sensor to measure a signal associated with a biological parameter (for example mechanical, electrical/ionic or fluorescence/chemiluminescence.

### Mechanical Sensor

An organized tissue produced according to the invention is tethered to attachment points at either end of a culture vehicle (open system, closed cartridge module, etc.). One or both ends of the tissue attachment sites is/are connected to a force transducer instrument (e.g. Model 400A Series Force Transducer Systems, Aurora Scientific, Inc.) that is connected to an oscilloscope to be used for monitoring the readout. In another embodiment the organized tissue is grown around the force transducer instrument. In another embodiment the organized tissue is impaled by the force transducer instrument.

The addition of certain agents to the media or perfusate of the organized tissue results in a change in the dimensions, contractile state, contractile frequency or force generated of or by the organized tissue. This change is detected by the attached force transducer and read out on the oscilloscope or a comparable apparatus.

This system can detect a range of frequencies from 0.5Hz to 100kHz, a change in dimensions in the range of approximately 0.1µm to 1cm and a change in force in the range of approximately 0.001 µg to 10,000 g.

An apparatus capable of mechanically stimulating the organized tissue with a known force (0 .001 µg to 10.000 g), distance (0.1µm to 1cm) or frequency range (0.01Hz to 100kHz) may also be included in this system and used for measurement, calibration, etc.. purposes. An example of this type of apparatus is the Series 300B Lever Systems (Aurora Scientific, Inc., Ontario, Canada).

### Electrical/Ionic Sensor

An organized tissue produced according to the invention is tethered to attachment points at either end of a culture vehicle (open system, closed cartridge module, etc.). One or both ends of the tissue attachment sites are connected to an electrical/ionic output measuring instrument that is connected to an oscilloscope to be used for monitoring the readout. In another embodiment the organized tissue is grown around the electrical/ionic output measuring instrument. In another embodiment the organized tissue is impaled by the electrical/ionic output measuring instrument.

The addition of certain agents to the media or perfusate of the organized tissue will result in a change in the electrical output of the organized tissue. This change will be detected by either attached surface EMG electrodes or an attached force transducer and read out on the oscilloscope or a comparable apparatus. The range of electrical output detected is from 1µV to 1000mV.

An apparatus capable of mechanically stimulating the organized tissue with a known force (0.001 µg to 10,000 g), distance (0.1µm to 1cm) or frequency range (0.01Hz to 100kHz) may also be included in this system and used for measurement, calibration, etc.. purposes. An example of this type of apparatus is the Series 300B Lever Systems (Aurora Scientific, Inc., Ontario, Canada).

### Fluorescent/Chemiluminescent Sensor

An organized tissue produced according to the invention, from cells transfected with a vector expressing an autofluorescent marker, for example the Green Fluorescent Protein (GFP), is connected to a light source in an instrument capable of measuring fluorescence. If a secreted form of the fluorescent maker is used, constant real-time marker production can be measured directly in the culture medium. If the marker is expressed intracellularly, the incident light beam is aimed directly at the organized tissue. The amount of fluorescent marker is quantitated by fluorescence using a multiwell plate fluorescence unit in which the tissues are grown.

Alternatively, an organized tissue is produced according to the invention, from cells stably transfected with a vector expressing secreted alkaline phosphatase (SEAP). The amount of secreted SEAP is measured by fluorescence or chemiluminescence in an aliquot of the culture medium following the addition of the chemiluminescent substrates CSPD or MUP. Alternatively, if the presence of the substrates is not detrimental to the cultured tissues, these substrates are added directly into the culture medium contained in the culture wells, and the amount of secreted SEAP measured by fluorescence or chemiluminescence.

### IN VIVO SCREENING METHODS

An in vivo screening method includes administering an exogenously produced candidate bioactive compound to a host organism in which an organized tissue is implanted and measuring in a subset of cells, in a single cell or in a body fluid or tissue of the host, a biological parameter modified by the candidate bioactive compound. The subset of cells, the entire organized tissue, or a body fluid or tissue of the host organism may optimally be removed prior to measuring a biological parameter

An additional method for in vivo screening includes producing an organized tissue that is comprised of cells genetically engineered to produce a candidate marker compound. Following implantation of the organized tissue into the host, the organized tissue will provide an endogenous source of a substance which is a measurable biological parameter to indicate bioactivity of a compound or will provide an endogenous source of the candidate marker compound itself to the host. A biological parameter that may be modified as a result of the implantation of the organized tissue and the endogenous production of a candidate marker compound will then be measured in a subset of cells, in a single cell or in the body fluids or tissue of the host.

An organized tissue is produced in vitro as described above. Implantation of the organized tissue into the host organism is then performed as follows.

### 1) In Vivo Screening in a Subset of Cells of the Implanted Organized Tissue

### Implantation

The organized tissue may be implanted by standard laboratory or surgical techniques at a desired anatomical location within the organism. For example, the organized tissue may be implanted in the same or a different tissue from the tissue of origin of at least one of the individual cells. The location of implantation depends, in part, upon the concentration, location and the identity of the particular compound to be detected. For example, an organized tissue acting as a screening organ for bioactive compounds may be implanted in or adjacent to a highly vascularized host tissue. Alternatively, an organized tissue acting as a screening organoid for muscle wasting is preferably implanted in or adjacent to the host muscle tissue to which the bioactive compound is to be delivered.

The organized tissue may be implanted by attachment to a host tissue or as a free floating tissue. Skeletal muscle organoids are preferably implanted by attachment to the host tissue under tension along a longitudinal axis of the organoid. Moreover, the organized tissue may be permanently or temporarily implanted. Furthermore, because organized tissue may be implanted, removed, and maintained *in vitro,* bioactive compounds may be delivered intermittently to the same or a different location in the organism. For example, a skeletal muscle organoid produced from the cells of a human patient (e.g., an autograft) may be implanted at a first anatomical location for a defined period and subsequently implanted at a second location at or after the time of removal.

Replicate samples of an organized tissue comprised of muscle cells (produced as described) are preferably implanted into at least two different host organisms. The effects of candidate biological compounds will be determined by comparing the level of a biological parameter in a subset of cells removed from the implanted organized tissue of an untreated host and a host treated with an exogenous source of a candidate bioactive compound.

### Administration

The route of administration of a candidate bioactive compound to the host organism may include oral consumption, or tissue absorption via topical compositions, suppositories, inhalants, or the like. Exogenous sources of the bioactive compound may also be provided continuously over a defined time period. For example delivery systems such as pumps, time-released compositions, or the like may be implanted into the organism on a semi-permanent basis for the administration of bioactive compounds (e.g. insulin, estrogen, progesterone, etc...). The compound can also be delivered to the host organism by implanting an organized tissue that has been genetically engineered to express the candidate bioactive compound into the host organism.

### Removing the Organized Tissue

The organized tissue may be removed from the host organism again according to standard surgical procedures. Alternatively, a cell or subset of cells of the organized tissue may be removed from the host organism

The organized tissue, cell, or "groups" of cells in the organized tissue are removed from the host by the following method.

All experimental animal procedures are approved by the Institutional Animal Care and Utilization Committee and conform to the guiding principles of the American Physiological Society. Before surgical removal of the organized tissue from, for example under the skin flap, the animals are anesthetized with a mixture of ketamine (55mg/kg), promazine (1 mg/kg) and xylazine (5mg/kg). The skin is shaved and sterilized. A 20-30 mm incision is made, the skin reflected, and organized tissue, cell or groups of cells in organized tissue, removed. The wound is then sutured closed. Removal of the organized tissue is a rapid procedure (<10 min).

### Measuring a Biological Parameter

Following removal of the organized tissue from the host where the organized tissue comprises muscle cells, muscle wasting will be measured in a subset of cells of the organized tissue by the in vitro assays described.

### 2) In Vivo Screening in a Cell of the Implanted Organized Tissue

Implantation of the organized tissue and administration of the candidate bioactive compound are as described above.

The measuring step of the inventive methods also may include measuring a biological parameter in a single cell, for example ion flux by microscopy, membrane potential, or the expression of Green Fluorescent Protein.

### 3) In Vivo Screening of the Body Fluids and Serum of the Host Organism Containing the Implanted Organized Tissue

Implantation of the organized tissue and administration of the candidate bioactive compound are as described. The measuring step also may include measuring a biological parameter in a body fluid of the host organism.

For an organized tissue comprising muscle cells, muscle atrophy in the organized tissue can be measured *in vivo* by the following method.

Alternatively, 3-methylhistidine can be measured in the urine of the animal by the following method (Auclair et al., Am. J. Physiol., 272: C1007-C1016) for measuring host muscle atrophy. Urinary N-acetylated 3-methylhistidine is hydrolyzed by a modification of the method of Lowell et al. (Lowell et al., Metabolism, 35: 1121-1127, 1986). Two ml of 4.5 N HCL is added to 2 ml of urine samples and heated in a boiling bath for two hours. Hydrolysates are neutralized with KOH, centrifuged, and the supernatants cleared by filtration through a 0.2µm cellulose filter and analyzed by HPLC as described (Fermo et al., J. Liquid Chromato., 14: 1715-1728, 1991 and Garrel et al., J. Parenter. Enteral Nut. 19: 482-491, 1995). With a flow rate of 0.5 ml per minute, 3-methylhistidine elutes at approximately 14 minutes. The concentration of 3-methylhistidine is determined by comparing peak areas of experimental samples to that of an external standard.

### 4) In Vivo Screening of a Candidate Bioactive Compound in a Host Transplanted with an Organized Tissue Genetically Engineered to Produce a Substance

An organized tissue comprised of muscle cells can be produced as described. Endogenous proteins are labeled by incubating the organized tissue in the presence of radiolabelled amino acids (for example ¹⁴C or ³H labeled phenylalanine or tyrosine) for 24 or more hours. Following implantation of the organized tissue into the animal, a candidate bioactive compound is administered as described above. Protein degradation resulting from muscle atrophy is quantitated by measuring the radioactive amino acids in the serum of the animal. Radiolabelled amino acids released into the serum are measured from the soluble fraction isolated after a TCA precipitation of the serum (Vandenburgh and Kaufman, supra).

Implantation of an organized tissue into a host organism provides for in vivo screening of compounds. Prior to implantation, the production of a candidate marker compound by a genetically engineered organized tissue may be measured and quantified per unit time, per unit mass, or relative to any other physiologically-relevant parameter. In addition, the capability of a genetically engineered organized tissue to sustain production of a candidate marker compound can be assessed by culturing for extended periods and assaying for compound production with time.

Moreover, because the organized tissue is implanted at a defined anatomical location as a discrete collection of cells, it may be distinguished from host tissues, removed post-implantation from the organism, and reimplanted into the organism at the same or a different location at the time of removal or following an interim period of culturing *in vitro*. This feature facilitates transient or localized secretion of the marker compound by the organoid. Restriction of the cells producing candidate marker compounds to particular anatomical sites also enhances the localized sensing to bioactive compounds. Likewise, the efficiency of implanting post-mitotic cells containing a foreign DNA sequence into an organism (i.e., the number of cells in a post-mitotic state as a percentage of the initial number of cells containing the foreign DNA sequence) is enhanced by organoid implantation as compared to the implantation of individual mitotic cells. For example, skeletal muscle organoids produced *in vitro* include post-mitotic myofibers representing greater than 70% of the initial myoblasts containing a foreign DNA sequence, whereas direct implantation of the myoblasts results in post-mitotic myofibers representing less than 1% of the initial cells.

To produce an organized tissue capable of sensing a candidate bioactive compound delivered to a host organism, muscle cells (e.g. C2C12) are stably transduced with expression vectors containing the gene for one of the candidate sensing compounds. Cells will be screened for sensitivity to the candidate bioactive compound. Cells expressing the marker compound will be expanded and used to produce an organized tissue.

At least two host organisms will be implanted with a control organized tissue comprised of untransfected cells. An additional host organism will be implanted with the organized tissue comprised of cells producing the candidate marker compound. Implantation will be as described. The candidate bioactive compound will be administered (as described) to one of the host organisms implanted with the control organized tissue. The activity of the candidate bioactive compound will be compared in these three host organisms by the in vitro or in vivo screening assays described.

### Candidate Compounds

In general, candidate compounds screened according to the invention could include but are not limited to toxins, cytokines, neurotransmitters, growth factors, morphogens, inhibitors, stimulators, bacteria, viruses, DNA, anti-sense nucleic acids, drugs, peptides and natural compounds. In particular embodiments, candidate compounds would be insulin-like growth factor, glucocorticoids or neurotropic factors. It is known that the ATP-dependent proteolytic pathway of protein degradation is activated during skeletal muscle atrophy. Other potential candidate bioactive compounds could include inhibitors of this degradative pathway including multipain inhibitors (sulfhydryl blocking agents), cystatin a (as well as other members of the cystatin family), peptide chloromethylketones, N-ethylmaleimide, hemin, analogs or derivatives of the thiol protease E64, peptide diazomethanes, isocoumarins, synthetic beta-lactams and the naturally occurring 40kDa proteasome inhibitor.

### KITS

Organized tissue-containing kits are also useful according to the invention. For example, a kit that includes a plurality (i.e., at least 6, preferably 24, 48, 96, and even up to several thousand) of organized tissues individually contained in a container that permits culture conditions in which the organized tissue is viable long term is particularly useful according to the invention. Minimally, the container will contain physiological media that permits viability of the tissue for storage and/or shipment purposes. Desirably, the medium and container will permit long-term viability and sampling of the organized tissue as described (above).

"Physiological" medium refers to any physiological solutions of salts and nutrients that permits maintenance of the tissue for at least 15 days, and shipment of the organized tissue; for example a medium for long term viability of the organized tissue will consist of DMEM with high glucose, 10% horse serum, 5% fetal calf serum, and 100 units/ml penicillin.

### Use and Administration

Candidate bioactive compounds identified according to the invention are potentially useful in treating disease involving a given tissue. Such compounds, once identified and tested for efficacy, may be delivered systemically or locally to an organism by a wide variety of methods. For example, an exogenous source (i.e. produced outside the organism treated) of the bioactive compound may be provided intermittently by repeated doses. For treatment, the route of administration may include oral consumption, injection, or tissue absorption via topical compositions, suppositories, inhalants, or the like. Exogenous sources of the bioactive compound may also be provided continuously over a defined time period. For example delivery systems such as pumps, time-released compositions, or the like may be implanted into the organism on a semi-permanent basis for the administration of bioactive compounds (e.g. insulin, estrogen, progesterone, etc...). Efficacy of the compound in disease treatment is indicated by amelioration or prevention of disease symptoms or the disease itself. The invention can also be used for screening potential biological and chemical toxins.

### OTHER EMBODIMENTS

Other embodiments will be evident to those of skill in the art. It should be understood that the foregoing detailed description is provided for clarity only and is merely exemplary.

## Claims

1. An *in vitro* method of screening a compound for bioactivity, comprising
contacting a candidate bioactive compound with an organized tissue, and
measuring in at least a cell of the organized tissue a biological parameter that is associated with bioactivity, wherein a change in the biological parameter that occurs as a result of said contacting step is indicative of bioactivity of said candidate compound.

2. The method of claim 1 wherein said contacting step comprises contacting a candidate bioactive compound with an organized tissue comprising substantially post-mitotic cells.

3. The method of claim 1 wherein said contacting step comprises contacting a candidate bioactive compound with an organized tissue comprising cells aligned substantially parallel to each other and along a dimension of the vessel in which the cells were grown.

4. The method of claims 1-3 wherein at least a subset of cells of said organized tissue contain a foreign DNA sequence.

5. The method of claim 4 wherein the cells containing the foreign DNA sequence produce a substance of a type that is not normally present in the cells or in an amount that is not normally produced by the cells.

6. The method of claim 1 wherein said contacting step comprises contacting a candidate bioactive compound with an organized tissue comprising muscle cells.

7. The method of claim 2 wherein said substantially post-mitotic cells are muscle cells.

8. The method of claim 3 wherein said cells aligned substantially parallel to each other are muscle cells.

9. The method of claim 6 wherein said biological parameter comprises protein degradation.

10. The method of claim 4 wherein said foreign DNA sequence is a reporter gene encoding a detectable protein.

11. The method of claim 10 wherein said measuring step comprises detecting said detectable protein.

12. A method of screening a compound for bioactivity, comprising
(A) administering a candidate bioactive compound to a non-human experimental animal in which an organized tissue is implanted wherein administration is selected from the group consisting of oral consumption, tissue absorption via topical compositions, via suppositories and via inhalants; and
(B) measuring a biological parameter that is associated with bioactivity in a urine or saliva sample of said non-human experimental animal, wherein a change in the biological parameter that occurs as a result of said contacting step is indicative of bioactivity of said candidate compound.

13. Use of a kit comprising a plurality of organoids wherein each organoid is individually contained in a physiological medium in a container for performing the method of claim 1 or 12.

14. The use of claim 13 and wherein said container is a well and said plurality of organoids is contained in a plurality of wells of a culture plate wherein each well contains an organoid wherein the organoid is viable long term.

## Patentansprüche

1. *In vitro*-Verfahren zur Durchmusterung einer Verbindung hinsichtlich biologischer Aktivität, bei dem man einen Kandidaten für eine biologisch aktive Verbindung mit einem organisierten Gewebe in Kontakt bringt und in wenigstens einer Zelle des organisierten Gewebes einen biologischen Parameter mißt, der mit biologischer Aktivität in Zusammenhang steht, wobei eine Veränderung des biologischen Parameters, die in Folge der Stufe des Inkontaktbringens stattfindet, ein Anzeichen für biologische Aktivität der Kandidatenverbindung ist.

2. Verfahren nach Anspruch 1, wobei die Stufe des Inkontaktbringens umfaßt, daß man einen Kandidaten für eine biologisch aktive Verbindung mit einem organisierten Gewebe in Kontakt bringt, welches im wesentlichen postmitotische Zellen enthält.

3. Verfahren nach Anspruch 1, wobei die Stufe des Inkontaktbringens umfaßt, daß man einen Kandidaten für eine biologisch aktive Verbindung mit einem organisierten Gewebe in Kontakt bringt, welches Zellen enthält, die im wesentlichen parallel zueinander und entlang einer Dimension des Gefäßes, in welchem die Zellen gezüchtet wurden, ausgerichtet sind.

4. Verfahren nach einem der Ansprüche 1 bis 3, wobei wenigstens eine Teilmenge der Zellen des organisierten Gewebes eine fremde DNA-Sequenz enthält.

5. Verfahren nach Anspruch 4, wobei die Zellen, welche die fremde DNA-Sequenz enthalten, einen Stoff von einer Art produzieren, der normalerweise nicht in den Zellen vorkommt, oder in einer Menge, die normalerweise von den Zellen nicht produziert wird.

6. Verfahren nach Anspruch 1, wobei die Stufe des Inkontaktbringens umfaßt, daß man einen Kandidaten für eine biologisch aktive Verbindung mit einem organisierten Gewebe, welches Muskelzellen enthält, in Kontakt bringt.

7. Verfahren nach Anspruch 2, wobei die im wesentlichen postmitotischen Zellen Muskelzellen sind.

8. Verfahren nach Anspruch 3, wobei die Zellen, die im wesentlichen parallel zueinander ausgerichtet sind, Muskelzellen sind.

9. Verfahren nach Anspruch 6, wobei der biologische Parameter Proteinabbau umfaßt.

10. Verfahren nach Anspruch 4, wobei die fremde DNA-Sequenz ein Reportergen ist, welches ein detektierbares Protein codiert.

11. Verfahren nach Anspruch 10, wobei die Stufe des Messens umfaßt, daß man das detektierbare Protein feststellt.

12. Verfahren zur Durchmusterung einer Verbindung hinsichtlich biologischer Aktivität, bei dem man
(A) einen Kandidaten für eine biologisch aktive Verbindung einem nicht menschlichen Versuchstier verabreicht, in welches ein organisiertes Gewebe implantiert ist, wobei die Verabreichung aus der Gruppe ausgewählt ist, bestehend aus oraler Aufnahme, Gewebeabsorption über topische Zusammensetzungen, über Suppositorien und über Inhalationsmittel, und
(B) in einer Urin- oder Speichelprobe des nicht menschlichen Versuchstieres einen biologische Parameter mißt, der mit biologischer Aktivität in Zusammenhang steht, wobei eine Veränderung in dem biologischen Parameter, die infolge der Stufe des Inkontaktbringens auftritt, ein Anzeichen für biologische Aktivität der Kandidatenverbindung ist.

13. Verwendung eines Kits, das eine Vielzahl von Organoiden umfaßt, wobei jedes Organoid einzeln in einem physiologischen Medium in einem Behälter enthalten ist, zur Durchführung des Verfahrens nach Anspruch 1 oder 12.

14. Verwendung nach Anspruch 13, wobei der Behälter ein Napf ist und die Vielzahl von Organoiden in einer Vielzahl von Näpfen in einer Kulturplatte enthalten sind, wobei jeder Napf ein Organoid enthält, wobei das Organoid über einen langen Zeitraum überlebensfähig ist.

## Revendications

1. Méthode *in vitro* pour sélectionner un composé pour une activité biologique, comprenant
la mise en contact d'un composé biologiquement actif candidat avec un tissu organisé, et
la mesure dans au moins une cellule du tissu organisé d'un paramètre biologique qui est associé à l'activité biologique, dans laquelle une variation du paramètre biologique qui se produit en résultat de ladite étape de mise en contact est une indication de l'activité biologique dudit composé candidat.

2. Méthode suivant la revendication 1, dans laquelle ladite étape de mise en contact comprend la mise en contact d'un composé biologiquement actif candidat avec un tissu organisé comprenant des cellules substantiellement post-mitotiques.

3. Méthode suivant la revendication 1, dans laquelle ladite étape de mise en contact comprend la mise en contact d'un composé biologiquement actif candidat avec un tissu organisé comprenant des cellules alignées substantiellement parallèlement les unes aux autres et le long d'une dimension du récipient dans lequel les cellules ont été cultivées.

4. Méthode suivant les revendications 1 à 3, dans laquelle au moins une sous-catégorie de cellules dudit tissu organisé contient une séquence d'ADN étrangère.

5. Méthode suivant la revendication 4, dans laquelle les cellules contenant la séquence d'ADN étrangère produisent une substance d'un type qui n'est pas normalement présent dans les cellules ou en une quantité qui n'est pas normalement produite par les cellules.

6. Méthode suivant la revendication 1, dans laquelle ladite étape de mise en contact comprend la mise en contact d'un composé biologiquement actif candidat avec un tissu organisé comprenant des cellules musculaires.

7. Méthode suivant la revendication 2, dans laquelle lesdites cellules substantiellement post-mitotiques sont des cellules musculaires.

8. Méthode suivant la revendication 3, dans laquelle lesdites cellules alignées substantiellement parallèlement les unes aux autres sont des cellules musculaires.

9. Méthode suivant la revendication 6, dans laquelle ledit paramètre biologique comprend la dégradation des protéines.

10. Méthode suivant la revendication 4, dans laquelle ladite séquence d'ADN étrangère est un gène rapporteur codant pour une protéine détectable.

11. Méthode suivant la revendication 10, dans laquelle ladite étape de mesure comprend la détection de ladite protéine détectable.

12. Méthode pour sélectionner un composé pour une activité biologique, comprenant
(A) l'administration d'un composé biologiquement actif candidat à un animal expérimental non humain dans lequel un tissu organisé est implanté, l'administration étant choisie dans le groupe consistant en la consommation orale, l'absorption tissulaire par l'intermédiaire de compositions topiques, par l'intermédiaire de suppositoires et par l'intermédiaire d'agents à inhaler ; et
(B) la mesure d'un paramètre biologique qui est associé à l'activité biologique dans un échantillon d'urine ou de salive dudit animal expérimental non humain, dans laquelle une variation dudit paramètre biologique qui se produit en résultat de ladite étape de mise en contact est une indication de l'activité biologique dudit composé candidat.

13. Utilisation d'un kit comprenant une pluralité d'éléments organoïdes, dans laquelle chaque élément organoide est présent individuellement dans un milieu physiologique dans un récipient pour la mise en oeuvre de la méthode de la revendication 1 ou 12.

14. Utilisation suivant la revendication 13 et dans laquelle ledit récipient est un puits et ladite pluralité d'éléments organoïdes est présente dans une pluralité de puits d'une plaque de culture, dans laquelle chaque puits contient un élément organoïde, ledit élément organoïde étant viable à long terme.
